# EUROPEAN PATENT SPECIFICATION

(11) **EP 4 101 411 B1**
(45) Date of publication and mention of the grant of the patent: **12.02.2025**
(21) Application number: 21913065.5
(22) Date of filing: 29.07.2021
(51) Int. Cl.: A61B 34/30, A61B 34/10, A61B 34/20, A61B 17/14, A61B 17/16, A61B 34/00, A61B 17/00

(54) **MECHANICAL ARM MOTION CONTROL SYSTEM AND SURGICAL OPERATION SYSTEM**
SYSTEM ZUR STEUERUNG DER BEWEGUNG EINES MECHANISCHEN ARMES UND CHIRURGISCHES BETRIEBSSYSTEM
SYSTÈME DE COMMANDE DU MOUVEMENT D'UN BRAS MÉCANIQUE ET SYSTÈME D'OPÉRATION CHIRURGICALE

(30) Priority: 30.12.2020 CN 202011631377
(43) Date of publication of application: 14.12.2022
(73) Proprietor: BEIJING HURWA ROBOT MEDICAL TECHNOLOGY CO., LTD., Beijing 100176 (CN); BEIJING HURWA ROBOT TECHNOLOGY CO., LTD., Beijing 100176 (CN)
(72) Inventor: LI, Shugang, Beijing 100176 (CN)
(74) Representative: Grey, Ian Michael
(86) International application number: PCT/CN2021/109410
(87) International publication number: WO 2022/142316

(56) References cited:
- CN-A- 111 182 847
- CN-A- 111 467 036
- CN-A- 112 890 954
- US-A1- 2013 006 267
- US-A1- 2017 231 701
- US-A1- 2019 142 518
- US-A1- 2020 093 552
- US-A1- 2020 138 534
- US-A1- 2020 383 734

## Description

### TECHNICAL FIELD

The present application relates to the technical field of surgical robots, and in particular, relates to a motion control method and system for a mechanical arm, and a surgical system.

### BACKGROUND

In a computer-assisted surgical system, operations are performed under assistance of a cooperative mechanical arm. A distal end of the collaborative mechanical arm generally carries an end effector. According to different situations, one end of the end effector carries different medical instruments, such as saws, drills, or milling cutters. Through the mechanical connection mechanisms and control operations among the cooperative mechanical arm, the end effector, and the medical instruments, the operations such as cutting, drilling or milling of the target are realized.

Taking the end effector carrying an electric oscillating saw for assisting osteotomy as an example, the process of a saw blade of the electric oscillating saw entering a standby position from any current position and entering an osteotomy position from the standby position, is called as position moving. After the mechanical arm moves until the saw blade is brought into the osteotomy position, the freedom of movement of the mechanical arm is limited in the osteotomy plane, so that the saw blade can only move in the locked plane; then the electric oscillating saw is powered on, and the doctor pushes the mechanical arm to control the saw blade in contact with the affected bone. After completing the osteotomy at one position, the saw blade of the electric oscillating saw moves to the next osteotomy position and the osteotomy is continued. During the above process, the monitor can display in real time the relative position between the digital three-dimensional models of the saw blade and the affected bone, as well as other prompt information for reference by professional physicians. The above operations are all completed by the professional physicians to ensure the control over the osteotomy. The professional physicians need to pay great attention when performing osteotomies to avoid cutting into soft tissue. For doctors who are not familiar with the operation or who have just completed the osteotomy for one time with high attention, they may need to think hard to think of the next operation to be performed on the mechanical arm, resulting in an unsmooth operation process and increased operation time.
US2019142518 A1 discloses a robotic system and method for robotic arthroplasty. The robotic system includes a machining station and a guidance station. The guidance station tracks movement of various objects in the operating room, such as a surgical tool, a humerus of a patient, and a scapula of the patient. The guidance station tracks these objects for purposes of controlling movement of the surgical tool relative to virtual cutting boundaries or other virtual objects associated with the humerus and scapula to facilitate preparation of bone to receive a shoulder implant system. In some versions, planning for placement of a stemless implant component is based on a future location of a stemmed shoulder implant to be placed in the humerus during a revision surgery.
US2017231701A1 discloses a device sized and shaped for insertion into a body, which includes at least one mechanical limb, including: a support segment; a first flexible section extending from the support segment and terminating in a coupling section; and a second flexible section extending from the coupling section and terminating in a tool or an connector for a tool; one or more of the flexible sections is bendable by at least 120°; a long axis length of the first flexible section is at least double a maximum extent of the first flexible section perpendicular to a flexible section long axis; a long axis length of the second flexible section is at least double a maximum extent of the second flexible section perpendicular to a flexible section long axis.
US2020138534A1 discloses a surgical robotic system, which includes an operating table, a plurality of robotic arms and surgical instruments, a user console, and a control tower. The plurality of robotic arms are mounted on the operating table and can be stowed folded under the table for storage. The user console has one or more user interface devices, which function as master devices to control the plurality of surgical instruments.

### SUMMARY

The invention is defined in the appended independent claim 1. Embodiments of the invention are defined in the dependent claims. The methods disclosed hereafter are exemplary only and do not form part of the invention.

The embodiments of the present disclosure provide a motion control method and system for a mechanical arm and a surgical system, which can improve the fluency of the surgical process.

On one aspect, a motion control method for a mechanical arm is provided, wherein a terminal end of the mechanical arm is adapted to carry an effector, and the method includes steps of: receiving a first control command from a first input device by a graphical user interface, wherein the first control command is configured to control the mechanical arm to move according to a first movement mode, and the first movement mode is that the mechanical arm moves from a current position to a target position under a driving of its own power; receiving a second control command from the first input device or a second input device, wherein the second control command is configured to control the mechanical arm to enter a second movement mode, and the second movement mode is that the mechanical arm is configured so that its terminal end can be translated in a predetermined plane and rotated about a normal line of the plane under a driving of an external force; and receiving a third control command from the second input device, wherein the third control command is configured to control the effector to perform a predetermined movement, wherein the first input device and the second input device are separated from each other or are provided to be separable from each other, and the first input device and the second input device are arranged within control ranges of different operators respectively.

According to the first aspect of the embodiments of the present application, the motion control method further includes a step of: inputting a fourth control command through the first input device or the second input device, wherein the fourth control command is configured to control the mechanical arm to stop moving according to the first movement mode.

According to the first aspect of the embodiments of the present application, the step of receiving the first control command from the first input device by the graphical user interface includes: receiving a pre-control command by the graphical user interface to provide a first control interface set, wherein the first control interface set is configured to receive the first control command from the first input device.

According to the first aspect of the embodiments of the present application, the method further includes a step of: after the execution of the third control command is completed, providing a first control interface set by the graphical user interface, wherein the first control interface set is configured to receive the first control command from the first input device.

On a second aspect, a motion control system for a mechanical arm is provided, a terminal end of the mechanical arm is adapted to carry an effector, and the system includes a controller provided with a graphical user interface, a first input device and a second input device, wherein: the graphical user interface is configured to receive a first control command from the first input device, the first control command is configured to control the mechanical arm to move according to a first movement mode, and the first movement mode is that the mechanical arm moves from a current position to a target position under a driving of its own power; the graphical user interface is further configured to receive a second control command from the first input device or the second input device, the second control command is configured to control the mechanical arm to enter a second movement mode, and the second movement mode is that the mechanical arm is configured so that its terminal end can be translated in a predetermined plane and rotated about a normal line of the plane under a driving of an external force; the second input device is configured to input a third control command, and the third control command is configured to control the effector to perform a predetermined movement; wherein the first input device and the second input device are separated from each other or are provided to be separable from each other, and the first input device and the second input device are arranged within control ranges of different operators respectively.

According to the second aspect of the embodiments of the present application, the first input device and the second input device are further configured to input a fourth control command, and the fourth control command is configured to control the mechanical arm to stop moving according to the first movement mode.

According to the second aspect of the embodiments of the present application, the graphical user interface is further configured to receive a pre-control command to provide a first control interface set, and the first control interface set is configured to receive the first control command from the first input device.

According to the second aspect of the embodiments of the present application, the graphical user interface is further configured to provide a first control interface set after the execution of the third control command is completed, and the first control interface set is configured to receive the first control command from the first input device.

According to the second aspect of the embodiments of the present application, the first input device is a voice input module, a mechanical button, a mechanical rocker or a wireless remote controller.

On a third aspect, a surgical system is provided, and the system includes a mechanical arm, a control system and a positioning system, the positioning system is configured to locate spatial orientation information of the mechanical arm, and the control system is configured to control a movement of the mechanical arm based on the spatial orientation information, wherein the control system is any motion control system for a mechanical arm on the second aspect.

In the above method and system, the first input device and the second input device can be arranged within the control ranges of different operators as required. For example, the first operator controls the first input device for input, and the second operator controls the second operation device for input. This allows different characters to make different inputs to control the mechanical arm to perform different actions at different stages. Each character can only focus on the input required by itself, and does not need to be familiar with the input operation of the whole process, thus providing a possibility to improve the fluency of the process.

### BRIEF DESCRIPTION OF THE DRAWINGS

The features, advantages and technical effects of the exemplary embodiments of the present application will be described below with reference to the accompanying drawings. In the accompanying drawings, the same components are given the same reference numerals. The accompanying drawings are not drawn to actual scale.
Fig. 1 is a schematic diagram of a surgical operation using a mechanical arm;
Fig. 2 is a flowchart of a motion control method for a mechanical arm provided by embodiments of the present application;
Fig. 3 is a schematic diagram of a graphical user interface provided by embodiments of the present application;
Fig. 4 is a schematic structural diagram of a motion control system for a mechanical arm provided by embodiments of the present application; and
Fig. 5 is a schematic structural diagram of a second input device according to embodiments of the present application.

### Description of reference numbers:

P-operating table, AM-mechanical arm, E-end effector, K-oscillating saw, A-control computer, I-image display device, V0-third interface, V1-first interface, V2-second interface;
10-first input device; 20-second input device, 21-upper left key, 22-upper right key, 23-main key; 30-controller, 31-graphical user interface; and
0, 1, 2, 3, 4, 5, 6 - tool position selection buttons.

### DETAILED DESCRIPTION

Features and exemplary embodiments of various aspects of the present application are described in detail below. In the following detailed description, numerous specific details are set forth in order to provide a thorough understanding of the present application. However, it is apparent to the person skilled in the art that the present application may be practiced without some of these specific details. The following description of the embodiments is merely to provide a better understanding of the present application by illustrating examples of the present application. In the accompanying drawings and the following description, at least some well-known structures and techniques are not shown in order to avoid unnecessarily obscuring the present application; and the dimensions of some structures may be exaggerated for clarity. Further, the features, structures, or characteristics described below may be combined in any suitable manner in one or more embodiments.

It should be noted that, in the present application, relational terms such as first and second are only used to distinguish one entity or operation from another entity or operation, and do not necessarily require or imply any actual relationship between these entities or operations or any sequence among them. Moreover, the terms "comprise", "include" or any other variations thereof are intended to encompass a non-exclusive inclusion such that a process, method, article or device that includes a list of elements includes not only these listed elements, but also includes other elements not explicitly listed or elements inherent to the process, method, article or device. Without further limitations, an element defined by the phrase "comprises" does not preclude the presence of additional identical elements in the process, method, article, or device that includes said element.

The present application provides a motion control method for a mechanical arm, which can optimize the operation process. A terminal end of the mechanical arm is adapted to carry an effector, the effector may be an electric driving device and can carry a tool such as a drill or a saw. The motion control method includes steps of: receiving a first control command from a first input device by a graphical user interface, wherein the first control command is configured to control the mechanical arm to move according to a first movement mode; receiving a second control command from a first input device or a second input device by the graphical user interface, wherein the second control command is configured to control the mechanical arm to enter a second movement mode; and inputting a third control command through the second input device, wherein the third control command is configured to control the effector to perform a predetermined movement, and the first input device and the second input device are separated from each other or are provided to be separable from each other.

In the above control method, the first input device and the second input device may be arranged within control ranges of different operators as required. For example, the first operator controls the first input device for input, and the second operator controls the second input device for input. This allows different characters to make different inputs, so as to control the mechanical arm to perform different actions at different stages, and each character can only focus on the input required by itself, without the need to be familiar with the input operations of the whole process, thereby providing a possibility to improve the fluency of the process. Moreover, the existing graphical user interface can be used to provide an input interface, and the operator can refer to the text and graphic prompts of the graphical user interface during the input process, thereby facilitating the accurate input.

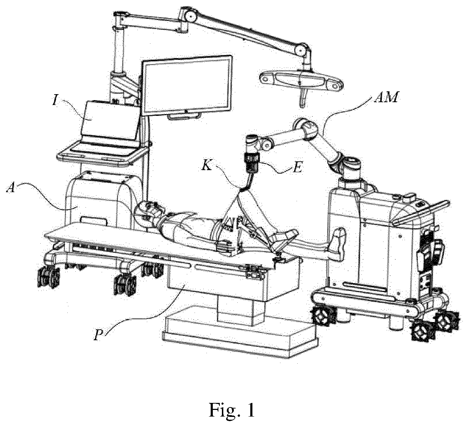

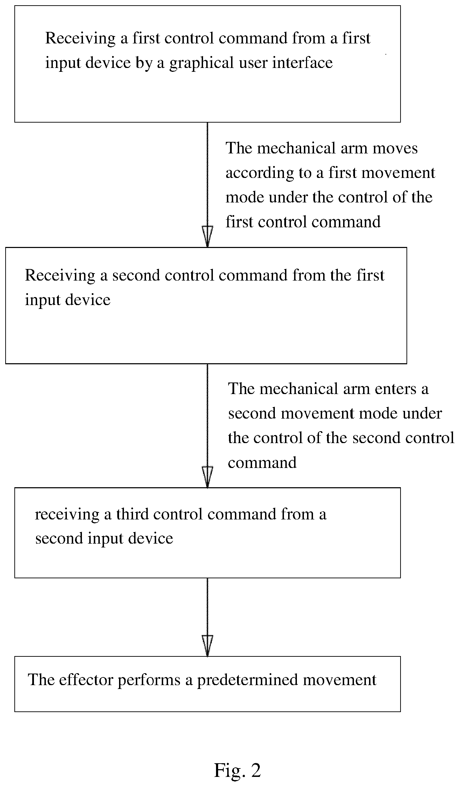

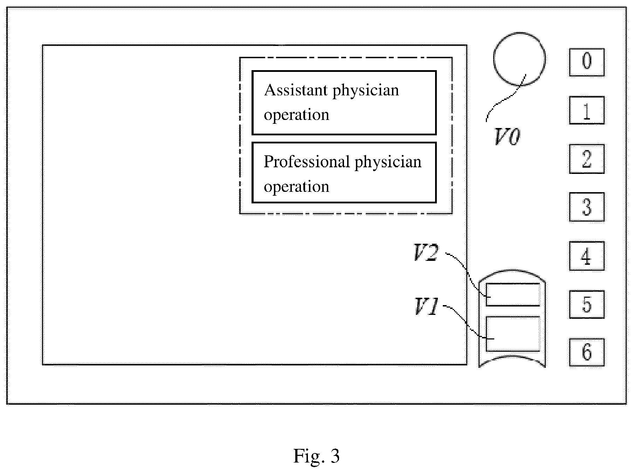

Referring to Fig. 1 to Fig. 3, Fig. 1 shows a schematic diagram of a surgical operation using a mechanical arm, Fig. 2 shows a flowchart of a motion control method for a mechanical arm provided by embodiments of the present application, and Fig. 3 is a schematic diagram of a graphical user interface provided by the embodiments of the present application.

The motion control method for a mechanical arm provided by the embodiments of the present application can be applied to a mechanical arm of a surgical system, and for the convenience of description, the embodiments of the present application take a total knee arthroplasty surgery as an example for description.

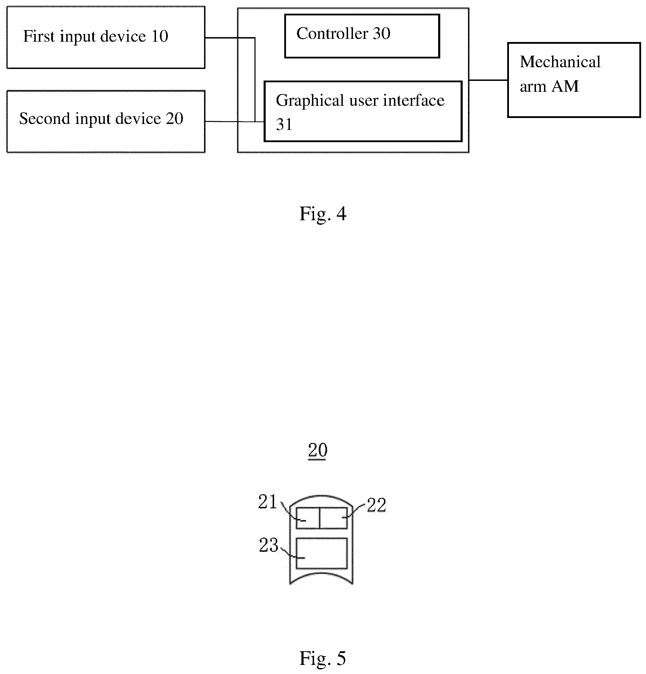

As shown in Fig. 1, the patient is lying on an operating table P, a terminal end of a mechanical arm AM is equipped with an end effector E, and a distal end of the end effector E is equipped with an oscillating saw K, which can perform cutting operations. The terminal end of the mechanical arm AM is located near the patient's affected part. A control computer A is arranged at a position further away from the operating table P, and the control computer A includes an image display device I and input devices such as a keyboard and mouse, for displaying required information and providing a graphical user interface. The first operator may stand at a position beside the operating table P, and also beside the mechanical arm AM to directly touch and operate the mechanical arm. An input device (not shown in the figure) can be arranged near the position where the first operator stands, and the input device can be a second input device 20 as shown in Fig. 5, which may be a mechanical foot operating device, and may include a main button 23, an upper left button 21 and an upper right key 22. The second input device 20 is communicably connected with the control computer A via a cable. A second operator can be beside the control computer A and operate the control computer A in combination with the graphical user interface displayed by the display device I.

The total knee arthroplasty surgery generally requires six osteotomies at different positions on the knee joint, and before each osteotomy, the oscillating saw K needs to be coincident with a planned osteotomy plane. In some alignment methods, the mechanical arm is manually dragged to make the oscillating saw K coincide with the planned osteotomy plane. Specifically, the surgical system can obtain in real time a relative positional relationship between the oscillating saw K and the planned osteotomy plane and can visualize the relative positional relationship between the two (for example, display it in a digital three-dimensional model). The mechanical arm is manually dragged to move, and an orientation of the mechanical arm is adjusted with reference to the relative positional relationship displayed on the display device. After the display device shows that the alignment is realized, the mechanical arm is manually locked at the current position.

In the control method for a mechanical arm provided by the present application, the mechanical arm receives the first control command input through the first input device and moves according to the first movement mode, that is, the mechanical arm automatically moves from an initial position to an osteotomy position by means of its own power and navigation of the system navigation. After arriving at the osteotomy position, the mechanical arm receives the second control command from the second input device and enters a second movement mode, that is, a moving range of the terminal end of the mechanical arm is limited within one plane, the movement manner of the mechanical arm merely includes translation within this plane and rotation around a normal line of this plane, and the mechanical arm can be moved according to the above-mentioned movement manner when driven by an external force (such as the operator's push and pull).

Specifically, the first input device may be a mouse or a keyboard, which may be arranged beside the control computer A as shown in Fig. 1 and operated by the first operator. The first operator may be a physician assistant, a nurse, or an equipment controller. The first control command can be input to the control computer through a human-computer interaction interface, and then be sent to the mechanical arm by the control computer. More specifically, the control computer is an upper computer, the mechanical arm is directly controlled by an industrial computer as a lower computer, the first control command is sent by the upper computer to the lower computer, and the lower computer controls the mechanical arm to move. The graphical user interface may include a first control interface set as shown in Fig. 3, and the first control interface set includes: a first interface V1 and a second interface V2. The first interface V1 and the second interface V2 are virtual buttons, and a cursor controlled by the mouse can click or hold down the virtual buttons to input commands, and predetermined control commands correspond to the operations of clicking or holding down. For example, the command corresponding to the operation "hold down the first interface V1" is the first control command, the movement corresponding to this command is that the terminal end of the mechanical arm AM moves according to a predetermined path, and on this basis, the operation "release the first interface V1" corresponds to a fourth control command of "stop moving"; or, the movement corresponding to the control command which corresponds to the operation "click the first interface V1" is that the terminal end of the mechanical arm AM moves according to a predetermined path, and on this basis, the control command corresponding to the operation "click the second interface V2" can control the mechanical arm to stop the current movement.

The second input device may be a mechanical foot operating device, and the mechanical foot operating device includes a mechanical button. The second input device may be arranged beside the operating table P, to facilitate the person who controls the mechanical arm AM to operator with the foot thereof. When the mechanical arm AM reaches the osteotomy position, it receives the second control command input through the second input device, and enters the second movement mode. The person who operates the second input device to perform the input operation may be the person who directly pushes the mechanical arm AM, such as the chief surgeon. The specific operation may be to click a button on the mechanical foot operating device by foot, such as the upper left key 21 as shown in Fig. 5. In some optional embodiments, the graphical user interface receives the control command input through the first input device and controls the mechanical arm to enter the second movement mode. For example, as shown in Fig. 3, the mechanical arm AM is made to move according to the first movement mode by holding down the first interface V1 with the cursor manipulated by the mouse; after the mechanical arm AM is in place, the second interface V2 is clicked by manipulating the cursor with the mouse, and the control computer receives the control command corresponding to this operation and controls the mechanical arm AM to enter the second movement mode.

After the operator holds on the mechanical arm AM, the mechanical arm receives the third control command input through the second input device, and the effector E starts to perform a predetermined action, wherein the predetermined action is that the oscillating saw K starts to oscillate according to a predetermined frequency and amplitude. The person who operates the second input device to input the third control command may be the person who directly pushes the mechanical arm AM, such as the chief surgeon. The specific operation can be stepping on a button of the mechanical foot operating device, such as the main key 23 as shown in Fig. 5. When the main key 23 is released, the control computer A can detect an electrical signal of this operation and send the control command corresponding to the signal to the mechanical arm AM, so as to control the effector E to stop the above-mentioned predetermined action. In some optional embodiments, the first control interface set is temporary, that is, it only appears when predetermined conditions are satisfied. For example, the first control interface appears only when the control computer receives a pre-control command. The pre-control command can also be received by the graphical user interface; as shown in Fig. 3, the graphical user interface includes a third interface V0, a first pre-control command is input by clicking the third interface V0 with the mouse cursor, and a new control interface is generated in the graphical user interface, for example, optional buttons of "assistant physician operation" and "professional physician operation". Then, a second pre-control command can be input by clicking the button of "assistant physician operation" through the mouse cursor, to call up the first interface V1 and the second interface V2. In some other optional embodiments, the pre-control command may further be a signal that the execution of the third control command ends. In some other optional embodiments, when the mechanical arm executes the first control command, the end effector is configured to be unable to execute the third control command, so as to prevent the end effector from being turned on when the mechanical arm moves and causing danger.

The navigation system includes a mechanical arm tracker arranged on the mechanical arm, a patient tracker arranged on the patient's body and a localizer. The patient tracer can indicate the orientation information of the planned osteotomy plane, the mechanical arm tracer can indicate the orientation information of the oscillating saw K, and the localizer can obtain the above-mentioned orientation information and transmit it to the control computer. The control computer controls the mechanical arm to move toward the osteotomy plane according to the predetermined path, and continuously compares the relative orientation relationship between the mechanical arm and the osteotomy plane during the movement of the mechanical arm until they coincide. The tracer includes at least three light sources with predetermined relative positions, and the light sources can be active light sources (such as light-emitting diodes) or passive light sources (such as infrared reflective balls). The localizer can be a binocular camera, which can receive a light signal of the tracer and locate spatial orientation information of the tracer.

Referring to Fig. 4 and Fig. 5, Fig. 4 is a schematic structural diagram of a motion control system for mechanical arm provided by embodiments of the present application, and Fig. 5 is a schematic structural diagram of a second input device provided by embodiments of the present application.

The motion control system for a mechanical arm is configured to control a movement of the mechanical arm, and a terminal end of the mechanical arm is adapted to carry an effector. The motion control system for a mechanical arm includes a controller 30 provided with a graphical user interface 31, a first input device 10 and a second input device 20. The graphical user interface 31 is configured to receive a first control command from the first input device 10, and the first control command is configured to control the mechanical arm to move according to a first movement mode; the graphical user interface 31 is further configured to receive a second control command from the first input device 10 or the second input device 20, and the second control command is configured to control the mechanical arm to enter a second movement mode; the second input device 20 is configured to input a third control command, and the third control command is configured to control the effector to perform a predetermined movement; the first input device 10 and the second input device 20 are separated from each other, and can be respectively arranged in different positions in the operating room.

The first input device 10 can be a mouse, the corresponding cursor of the mouse can be displayed in the graphical user interface 31, and the first input device 10 may be arranged near the image display device 1 or the control computer A in Fig. 1.

The second input device 20 can be the mechanical foot operating device as shown in Fig. 5, can be communicatively connected with the controller 30 through a cable, and can be arranged around the operating table P.

The controller 30 is the control computer A or a control unit in the control computer A, and the graphical user interface 31 is an input and output interface of the control computer A, which is presented by the image display device I. The controller 30 may output control commands to the mechanical arm AM.

The input manners of the first control command, the second control command, the third control command and the fourth control command may be consistent with the input manners mentioned in the foregoing embodiments of the control method, and the movement manners of the mechanical arms corresponding to the respective control commands may also refer to the description in the foregoing embodiments.

In some optional embodiments, the first input device 10 can further be used to input the second control command.

In some optional embodiments, the first input device 10 further may be a voice input module. The operator can input voice commands to the controller 30 through the voice input module, the graphical user interface 31 receives the corresponding commands and generates one or more corresponding input interfaces (such as virtual buttons), and then the desired input interface can be selected by further voice commands or other input devices, so as to complete the input. The voice input module can be arranged near the control computer A to facilitate the input by the person who operates the control computer A or arranged at other positions for input by a predetermined person.

In some other optional embodiments, the first input device may be a mechanical button, a mechanical rocker, or a wireless remote controller, and may be provided at any position.

In some other optional embodiments, the second input device may be a mechanical rocker, a mechanical button or a wireless remote controller, and the second input device may be provided on the mechanical arm, beside an operating bed or on the floor of an operating room, as long as it is convenient for the attending physician who operates the mechanical arm to operate the second input device.

Examples of the specific steps of using the control system or the control method please refer to the following description: as shown in Fig. 3, the cutter position selection button 0 is the standby cutter position selection button, and when this button is selected by being clicked with the mouse cursor or an command input through other input devices, the terminal end of the mechanical arm can be moved to a preset position. The cutter position selection buttons 1 to 6 are selection buttons corresponding to 6 osteotomy positions in the knee arthroplasty surgery. During the operation, the third interface V0 can be clicked first so that the selection buttons of "Operation by Assistant Physician" and "Operation by Professional Physician" appear in the graphical user interface, and then the button of "Operation by Professional Physician" is selected so that the first interface V1 and the second interface V2 appear in the graphical user interface. A desired osteotomy position is selected, for example, the cutter position selection button 1 is clicked, and then the first interface V1 is held on by the mouse, so that the mechanical arm moves from the standby cutter position to the osteotomy position corresponding to the cutter position selection button 1 according to the predetermined path in the first movement mode. After the mechanical arm arrives at the position, the second interface V2 is clicked, and the corresponding control command makes the mechanical arm enter the second movement mode, then, the doctor can hold on the mechanical arm and step on the main button 23 in the second input device as shown in Fig. 5, so that the oscillating saw K of the end effector E is oscillated, and the doctor pushes the mechanical arm to move to start the osteotomy.

The present application further provides a surgical system, which includes a mechanical arm, a control system, and a positioning system, the positioning system is configured to locate spatial orientation information of the mechanical arm, and the control system is configured to control the movement of the mechanical arm based on the spatial orientation information, wherein the control system is the motion control system for a mechanical arm described in any one of the above-mentioned embodiments.

The control method and system for a mechanical arm and the surgical system provided by the present application allow different characters to control the movement of the mechanical arm at different stages through different input devices, so that each character can only focus on the operations corresponding to their own roles without the need to be familiar with the entire operation process, which helps to improve the fluency of the operating process.

Although the present application has been described with reference to the preferred embodiments, various modifications may be made and equivalents may be used to replace the components therein without departing from the scope of the present application. Particularly, as long as there is no structural conflict, the various technical features mentioned in the various embodiments can be combined in any manner. The invention is not limited to the specific embodiments disclosed herein but includes all the technical solutions falling within the scope of the claims.

## Claims

1. A motion control system for a mechanical arm (AM), a terminal end of the mechanical arm (AM) is adapted to carry an effector (E), and the motion control system comprises a controller (30) provided with a graphical user interface (31), a first input device (10) and a second input device (20), **characterized in that**:
the graphical user interface (31) is configured to receive a first control command from the first input device (10), the first control command is configured to control the mechanical arm (AM) to move according to a first movement mode, and the first movement mode is that the mechanical arm (AM) moves from a current position to a target position under a driving of its own power;
the graphical user interface (31) is further configured to receive a second control command from the first input device (10) or the second input device (20), the second control command is configured to control the mechanical arm (AM) to enter a second movement mode, and the second movement mode is that the mechanical arm (AM) is configured so that its terminal end can be translated in a predetermined plane and rotated about a normal line of the plane under a driving of an external force;
the second input device (20) is configured to input a third control command, and the third control command is configured to control the effector (E) to perform a predetermined action, the predetermined action is that an oscillating saw (K) carried by the effector (E) starts to oscillate according to a predetermined frequency and amplitude;
wherein the first input device (10) and the second input device (20) are separated from each other or are provided to be separable from each other, and the first input device (10) and the second input device (20) are arranged within control ranges of different operators respectively, and
the effector (E) is configured to be unable to execute the third control command when the mechanical arm (AM) executes the first control command.

2. The motion control system for a mechanical arm (AM) according to claim 1, **characterized in that**, the first input device (10) and the second input device (20) are further configured to input a fourth control command, and the fourth control command is configured to control the mechanical arm (AM) to stop moving according to the first movement mode.

3. The motion control system for a mechanical arm (AM) according to claim 1, **characterized in that**, the graphical user interface (31) is further configured to receive a pre-control command to provide a first control interface set, and the first control interface set is configured to receive the first control command from the first input device (10).

4. The motion control system for a mechanical arm (AM) according to claim 1, **characterized in that**, the graphical user interface (31) is further configured to provide a first control interface set after the execution of the third control command is completed, and the first control interface set is configured to receive the first control command from the first input device (10).

5. The motion control system for a mechanical arm (AM) according to any of claims 1 to 4, **characterized in that**, the first input device (10) is a voice input module, a mechanical button, a mechanical rocker or a wireless remote controller.

6. A surgical system comprising the motion control system for a mechanical arm according to any of claims 1 to 5, **characterized in that**, the surgical system further comprises a mechanical arm (AM), a control system and a positioning system, the positioning system is configured to locate spatial orientation information of the mechanical arm (AM), and the control system is configured to control a movement of the mechanical arm (AM) based on the spatial orientation information.

## Patentansprüche

1. Bewegungssteuerungssystem für einen mechanischen Arm (AM), wobei ein Terminalende des mechanischen Arms (AM) dazu eingerichtet ist, einen Aktuator (E) zu tragen, und das Bewegungssteuerungssystem eine Steuerung (30) umfasst, die mit einer grafischen Benutzeroberfläche (31), einer ersten Eingabevorrichtung (10) und einer zweiten Eingabevorrichtung (20) versehen ist, **dadurch gekennzeichnet, dass**:
die grafische Benutzeroberfläche (31) dazu konfiguriert ist, einen ersten Steuerbefehl von der ersten Eingabevorrichtung (10) zu empfangen, der erste Steuerbefehl dazu konfiguriert ist, den mechanischen Arm (AM) zum Bewegen gemäß einem ersten Bewegungsmodus zu steuern, und der erste Bewegungsmodus darin besteht, dass sich der mechanische Arm (AM) unter dem Antrieb seiner eigenen Kraft von einer aktuellen Position zu einer Zielposition bewegt;
die grafische Benutzerschnittstelle (31) ferner dazu konfiguriert ist, einen zweiten Steuerbefehl von der ersten Eingabevorrichtung (10) oder der zweiten Eingabevorrichtung (20) zu empfangen, wobei der zweite Steuerbefehl dazu konfiguriert ist, den mechanischen Arm (AM) zum Eintritt in einen zweiten Bewegungsmodus zu steuern, und der zweite Bewegungsmodus darin besteht, dass der mechanische Arm (AM) zur translatorischen Bewegung seines Terminalendes in einer vorbestimmten Ebene und zum Drehen des Terminalendes um eine Normale der Ebene unter dem Antrieb einer externen Kraft konfiguriert ist;
die zweite Eingabevorrichtung (20) dazu konfiguriert ist, einen dritten Steuerbefehl einzugeben, und der dritte Steuerbefehl dazu konfiguriert ist, den Aktuator (E) zum Ausführen einer vorbestimmten Aktion zu steuern, wobei die vorbestimmte Aktion darin besteht, dass eine von dem Aktuator (E) getragene oszillierende Säge (K) gemäß einer vorbestimmten Frequenz und Amplitude zu oszillieren beginnt;
wobei die erste Eingabevorrichtung (10) und die zweite Eingabevorrichtung (20) voneinander getrennt sind oder voneinander trennbar vorgesehen sind, und die erste Eingabevorrichtung (10) und die zweite Eingabevorrichtung (20) jeweils innerhalb von Steuerbereichen unterschiedlicher Bediener angeordnet sind, und
der Aktuator (E) so konfiguriert ist, dass er den dritten Steuerbefehl nicht ausführen kann, wenn der mechanische Arm (AM) den ersten Steuerbefehl ausführt.

2. Bewegungssteuerungssystem für einen mechanischen Arm (AM) nach Anspruch **1, dadurch gekennzeichnet, dass** die erste Eingabevorrichtung (10) und die zweite Eingabevorrichtung (20) ferner dazu konfiguriert sind, einen vierten Steuerbefehl einzugeben, und der vierte Steuerbefehl dazu konfiguriert ist, den mechanischen Arm (AM) zum Anhalten der Bewegung gemäß dem ersten Bewegungsmodus zu steuern.

3. Bewegungssteuerungssystem für einen mechanischen Arm (AM) nach Anspruch **1, dadurch gekennzeichnet, dass** die grafische Benutzerschnittstelle (31) ferner dazu konfiguriert ist, einen Vorsteuerbefehl zu empfangen, um einen ersten Steuerschnittstellensatz bereitzustellen, und der erste Steuerschnittstellensatz dazu konfiguriert ist, den ersten Steuerbefehl von der ersten Eingabevorrichtung (10) zu empfangen.

4. Bewegungssteuerungssystem für einen mechanischen Arm (AM) nach Anspruch **1, dadurch gekennzeichnet, dass** die grafische Benutzerschnittstelle (31) ferner dazu konfiguriert ist, einen ersten Steuerschnittstellensatz bereitzustellen, nachdem die Ausführung des dritten Steuerbefehls abgeschlossen ist, und der erste Steuerschnittstellensatz dazu konfiguriert ist, den ersten Steuerbefehl von der ersten Eingabevorrichtung (10) zu empfangen.

5. Bewegungssteuerungssystem für einen mechanischen Arm (AM) nach einem der Ansprüche 1 bis 4, **dadurch gekennzeichnet, dass** die erste Eingabevorrichtung (10) ein Spracheingabemodul, ein mechanischer Taster, ein mechanischer Joystick oder eine drahtlose Fernbedienung ist.

6. Chirurgisches System mit dem Bewegungssteuerungssystem für einen mechanischen Arm nach einem der Ansprüche 1 bis 5, **dadurch gekennzeichnet, dass** das chirurgische System ferner einen mechanischen Arm (AM), ein Steuerungssystem und ein Positionierungssystem umfasst, wobei das Positionierungssystem dazu konfiguriert ist, räumliche Orientierungsinformationen des mechanischen Arms (AM) zu lokalisieren, und das Steuerungssystem dazu konfiguriert ist, eine Bewegung des mechanischen Arms (AM) basierend auf den räumlichen Orientierungsinformationen zu steuern.

## Revendications

1. Système de commande de mouvement pour un bras mécanique (AM), dans lequel une extrémité terminale du bras mécanique (AM) est adaptée pour porter un actionneur (E), et le système de commande de mouvement comprend un contrôleur (30) pourvu d'une interface graphique d'utilisateur (31), un premier dispositif d'entrée (10) et un deuxième dispositif d'entrée (20), **caractérisé en ce que** :
l'interface graphique d'utilisateur (31) est configurée pour recevoir une première instruction de commande depuis le premier dispositif d'entrée (10), la première instruction de commande est configurée pour commander le bras mécanique (AM) à se déplacer selon un premier mode de mouvement, et le premier mode de mouvement est que le bras mécanique (AM) se déplace à partir d'une position actuelle vers une position cible sous un entraînement de sa propre puissance ;
l'interface graphique d'utilisateur (31) est configurée en outre pour recevoir une deuxième instruction de commande à partir du premier dispositif d'entrée (10) ou du deuxième dispositif d'entrée (20), la deuxième instruction de commande est configurée pour commander le bras mécanique (AM) à passer à un deuxième mode de mouvement, et le deuxième mode de mouvement est que le bras mécanique (AM) est configuré pour que son extrémité terminale puisse effectuer une translation dans un plan prédéfini et effectuer une rotation autour d'une normal du plan sous un entraînement d'une force externe ;
le deuxième dispositif d'entrée (20) est configuré pour saisir une troisième instruction de commande, et la troisième instruction de commande est configurée pour commander l'actionneur (E) à effectuer une action prédéfinie, et l'action prédéfinie est qu'une scie oscillante (K) portée par l'actionneur (E) commence à osciller selon une fréquence et une amplitude prédéfinies ;
dans lequel le premier dispositif d'entrée (10) et le deuxième dispositif d'entrée (20) sont séparés l'un de l'autre ou sont prévus pour être séparables l'un de l'autre, et le premier dispositif d'entrée (10) et le deuxième dispositif d'entrée (20) sont disposés dans des plages de commande de différents opérateurs respectivement, et
l'actionneur (E) est configuré pour être incapable d'exécuter la troisième instruction de commande lorsque le bras mécanique (AM) exécute la première instruction de commande.

2. Système de commande de mouvement pour un bras mécanique (AM) selon la revendication 1, **caractérisé en ce que**, le premier dispositif d'entrée (10) et le deuxième dispositif d'entrée (20) sont configurés en outre pour saisir une quatrième instruction de commande, et la quatrième instruction de commande est configurée pour commander le bras mécanique (AM) à arrêter de se déplacer selon le premier mode de mouvement.

3. Système de commande de mouvement pour un bras mécanique (AM) selon la revendication 1, **caractérisé en ce que** l'interface graphique d'utilisateur (31) est configurée en outre pour recevoir une instruction de pré-commande afin de fournir un premier ensemble d'interfaces de commande, et le premier ensemble d'interfaces de commande est configuré pour recevoir la première instruction de commande depuis le premier dispositif d'entrée (10).

4. Système de commande de mouvement pour un bras mécanique (AM) selon la revendication **1, caractérisé en ce que** l'interface graphique d'utilisateur (31) est configurée en outre pour fournir un premier ensemble d'interfaces de commande après l'exécution de la troisième instruction de commande, et le premier ensemble d'interfaces de commande est configuré pour recevoir la première instruction de commande depuis le premier dispositif d'entrée (10).

5. Système de commande de mouvement pour un bras mécanique (AM) selon l'une quelconque des revendications 1 à 4, **caractérisé en ce que** le premier dispositif d'entrée (10) est un module d'entrée vocale, un bouton mécanique, une bascule mécanique ou une télécommande sans fil.

6. Système chirurgical comprenant le système de commande de mouvement pour un bras mécanique selon l'une quelconque des revendications 1 à 5, **caractérisé en ce que**, le système chirurgical comprend en outre un bras mécanique (AM), un système de commande et un système de positionnement, le système de positionnement est configuré pour localiser des informations d'orientation spatiale du bras mécanique (AM), et le système de commande est configuré pour commander un mouvement du bras mécanique (AM) sur la base des informations d'orientation spatiale.
